(19) Europäisches Patentamt
European Patent Office
Office européen des brevets

(11) Publication number: **0 311 391 B1**

(12) **EUROPEAN PATENT SPECIFICATION**

(45) Date of publication of patent specification: **29.12.93**

(51) Int. Cl.5: **C07K 1/02**, C07K 7/06, //A61K37/02

(21) Application number: **88309306.4**

(22) Date of filing: **06.10.88**

(54) **New process for preparing an octapeptide.**

(30) Priority: **07.10.87 GB 8723484**

(43) Date of publication of application:
**12.04.89 Bulletin 89/15**

(45) Publication of the grant of the patent:
**29.12.93 Bulletin 93/52**

(84) Designated Contracting States:
**AT BE CH DE ES FR GB GR IT LI LU NL SE**

(56) References cited:

**No relevant documents have been disclosed.**

(73) Proprietor: **FARMITALIA CARLO ERBA S.r.L.**
**Via Carlo Imbonati 24**
**I-20159 Milano(IT)**

(72) Inventor: **Galantino, Mauro**
**Via dei Carracci, 8**
**Milan(IT)**
Inventor: **Forino, Romualdo**
**Via del Caravaggio, 14**
**Milan(IT)**
Inventor: **de Castiglione, Roberto**
**Via Domenichino, 38**
**Milan(IT)**

(74) Representative: **Woods, Geoffrey Corlett et al**
**J.A. KEMP & CO.**
**14 South Square**
**Gray's Inn**
**London WC1R 5EU (GB)**

EP 0 311 391 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a method for the preparation of the octapeptide of formula H-Leu-Glu-Asp-Gly-Pro-Lys-Phe-Leu-OH (A) and its pharmaceutically acceptable salts. The peptide, having thymic humoral activity, was originally isolated from calf thymus glands and then obtained by solid-phase synthesis, as described in US-A-4,621,135.

The present synthesis, based on conventional solution methods, offers the advantage of easier scale-up and better final yields for the purified target product. As a matter of fact, in the process of the present invention there is no formation of a succinimidyl derivative which is the main cyclic byproduct formed in the solid-phase synthesis of the prior art.

The byproduct is formed by an internal reaction between the $\beta$- carboxy group of Asp (even if protected) and the amino group of Gly. The reaction occurs in strongly acid or basic conditions, such as those employed for removing the final peptide from the solid support with hydrofluoric acid.

The synthetic scheme according to the invention can be represented as follows:

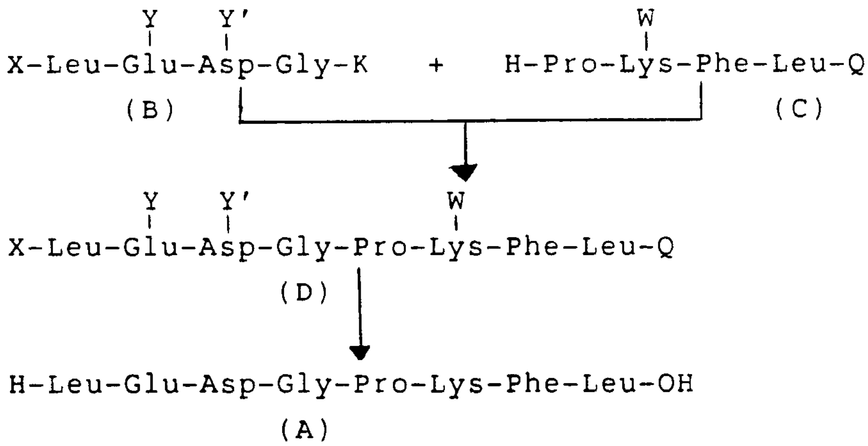

(B) (C) (D) (A)

wherein:

X and W independently represent amino protecting groups, K represents a hydroxy or hydrazido group, Q represents a carboxy protecting group or a hydroxy group with the proviso that Q must be a carboxy protecting group when K is a hydroxy group, and Y and Y' each independently represents a carboxy protecting group. The peptides (B) and (C) are condensed, the resultant peptide (D) is deprotected and, if desired, the resulting peptide (A) is converted into a pharmaceutically acceptable salt.

The peptides (B) and (C) and the resultant intermediate (D) may have their amino and carboxyl groups, which are not involved in the formation of the peptide linkage, blocked by suitable protecting groups. These protecting groups are removable by acidolysis, saponification, hydrogenolysis or other methods according to the methods known in peptide chemistry. The following groups may be used for the protection of the amino functions: benzyloxycarbonyl, t-butyloxycarbonyl, trityl, formyl, trifluoroacetyl, o-nitrophenylsulphenyl, 4-methoxybenzyloxy-carbonyl, 9-fluorenylmethoxycarbonyl, 3,5-dimethoxy-$\alpha$, $\alpha$'-dimethylbenzyloxycarbonyl and methylsulphonylethoxy-carbonyl. The following groups may be used for the protection of the carboxy functions: methyl, ethyl, t-butyl, benzyl, p-nitrobenzyl, 9-fluorenylmethyl, phenyl, 2,2,2-trichloroethyl and substituted hydrazides.

The condensation between the amino group of peptide (C) and carboxy group of peptide (B) to form the intermediate (D) may be carried out through an activated acyl-derivative such as a mixed anhydride, an azide, an activated ester, an imidazole or substituted imidazole derivative or by direct condensation between the free amino group and the carboxyl group, in the presence of a condensing agent such as dicyclohexyl carbodiimide alone or together with N-hydroxysuccinimide or 1-hydroxybenzotriazole or 1-hydroxybenzotriazole and 4-dimethylamino-pyridine. The activated acyl-derivative may be formed "in situ" starting from the compound (B) wherein K is OH or $NHNH_2$ using known methods.

The condensation may be carried out in a solvent such as dimethyl formamide, pyridine, acetonitrile, tetrahydrofuran, N-methyl-2-pyrrolidone and dimethylsulfoxide. The reaction temperature may be from -30 °C to ambient temperature. The reaction time is generally from 1 to 120 hours. Typically both Y and Y' groups comprise the same carboxy protecting group, Q represents a carboxy protecting group and K represents a hydroxy group.

The scheme of synthesis, the protecting groups and the condensing agents are selected so as to avoid the risk of racemization. The peptide of formula (A) or pharmaceutically acceptable salt thereof thus produced is recovered. It may be formulated with a pharmaceutically acceptable carrier or diluent to prepare a pharmaceutical composition for administration.

The preparation of starting materials (B) and (C) may be accomplished by known classical solution methods. A useful compound of formula (B) is the compound:

Z-Leu-Glu(OBzl)-Asp(OBzl)-Gly-OH (VIII)

wherein Z represents benzyloxycarbonyl and Bzl represents benzyl, which can been obtained by treating with zinc powder a compound of the formula (VII):

Z-Leu-Glu(OBzl)-Asp(OBzl)-Gly-OTce (VII)

wherein Z and Bzl are as defined above and Tce represents 2,2,2-trichloroethyl.

The following Examples illustrate the invention without limiting it. Rf values were determined on pre-coated plates of silica gel 60 $F_{254}$ (Merck), layer thickness 0.25 mm, length 20 cm, using the following development systems:

System A: n-butanol/acetic acid/water = 4/1/1 by volume.
System B: benzene/ethyl acetate/acetic acid/water = 10/10/2/1 by volume (upper phase).
System C: benzene/benzine (60-80)/ethyl acetate = 25/5/70 by volume.

"Merck" is a Trade Mark.

TLC analyses were carried out at a temperature ranging from 18°C to 25°C: the Rf values therefore can change by ± 5%. The Rt values were obtained from HPLC analyses using the following systems:

System 1 - Mobile phase: A (0,02 M $KH_2PO_4$, pH 3.3, buffer containing 10% v/v acetonitrile);
B (0,02 M $KH_2PO_4$, pH 3.3, buffer containing 60% v/v acetonitrile)
Column: μ BONDAPAK ™ $C_{18}$ (WATERS) 10 μm (3.9 mm x 30 cm)
Flow rate: 1 ml/min
U.V. detection: 210 nm.

System 2 - Mobile phase: A (0.02 M $KH_2PO_4$, pH 3.5, buffer containing 10% v/v acetonitrile);
B (0.02 M $KH_2PO_4$, pH 3.5, buffer containing 70% v/v acetonitrile)
Column: μ BONDAPAK ™ $C_{18}$ (WATERS) 10 μm (3.9 mm x 30 cm)
Flow rate: 1 ml/min
U.V. detection: 210 nm.

System 3 - Mobile phase: A (0.02 M $KH_2PO_4$, pH 3.5, buffer containing 10% v/v acetonitrile);
B (0.02 M $KH_2PO_4$, pH 3.5, buffer containing 70% v/v acetonitrile)
Column: RP-18 5 μm (4 mm x 25 cm) Lichrosorb (Merck)
Flow rate: 1 ml/min
U.V. detection: 210 nm.

System 4 - Mobile phase: A (0.02 M $KH_2PO_4$, pH 3.5, buffer containing 10% v/v acetonitrile);
B (0.02 M $KH_2PO_4$, pH 3.5, buffer containing 70% v/v acetonitrile)
Column: μ BONDAPAK ™ $C_{18}$ (WATERS) 10 μm (3.9 mm X 30 cm)
Flow rate: 2.0 ml/min
U.V. detection: 210 nm.

System 5 - Mobile phase: A (0.02 M $KH_2PO_4$, pH 3.3, buffer containing 10% v/v acetonitrile);
B (0.02 M $KH_2PO_4$, pH 3.3, buffer containing 60% v/v acetonitrile)
Column: μ BONDAPAK ™ $C_{18}$ (WATERS) 10 μm (3.9 mm X 30 cm)
Flow rate: 1.5 ml/min
U.V. detection: 210 nm.

Melting points were determined in open capillaries with a Tottoli apparatus and are uncorrected. Most derivatives soften and decompose before melting. In this specification symbols and abbreviations are those commonly used in peptide chemistry (see, e.g., Eur. J. Biochem., 138, 9-37 (1984)).

Other symbols and abbreviations used in the following Examples are: AcOH, glacial acetic acid; AcOEt, ethyl acetate; dec., decomposition; DCC, dicyclohexyl carbodiimide; DCEU, dicyclohexylurea; DMAP, 4-dimethyl amino-pyridine; DMF, dimethylformamide, ECC, ethyl chloroformate; $Et_2O$, diethyl ether; EtOH, ethyl alcohol 95%; HOBt, 1-hydroxybenzotriazole; i - $Pr_2O$, diisopropyl ether; i-PrOH, isopropyl alcohol; HPLC, high performance liquid chromatography; MeOH, methyl alcohol; NMM, N-methyl morpholine; Tce, 2,2,2-trichloroethyl; THF, tetrahydrofuran; TLC, thin layer chromatography.

EXAMPLE

Preparation of H-Leu-Glu-Asp-Gly-Pro-Lys-Phe-Leu-OH . 2HCl

Step 1 - Boc-Gly-OTce (I)

A solution of 3.85 g (22 mmoles) of BOC-Gly-OH and 3.16 ml (33 mmoles) of 2,2,2 trichloroethanol in DMF, are made to react for 18 hours at room temperature with 1.3 g (11 mmoles) of DMAP and 4.95 g (25 mmoles) of DCC. The reaction mixture was then filtered from DCEU and evaporated in vacuo.
The crude product was purified by column chromatography on silica gel (Merck) 0.040-0.063 mm, eluting with $CH_2Cl_2$ . 5.5 g (86% yield) of compound I were obtained as an oil: $Rf_c$ = 0.69.

Step 2 - HCl . H-Gly-OTce (II)

35.0 g (114.2 mmoles) of Boc-Gly-OTce (I) were dissolved in 350 ml of a saturated solution of HCl in AcOH. After 30 minutes at room temperature the Boc-removal was complete and the solvent was evaporated in vacuo at 30 °C. 24.0 g (90% yield) of compound II were obtained from AcOEt: mp 198-201 °C (dec.); $Rf_A$ = 0.70.

Step 3 - Boc-Asp(OBzl)-Gly-OTce (III)

To a solution of 37.7 g (116.50 mmoles) of Boc-Asp(OBzl)-OH in 500 ml of anhydrous THF, 12.9 ml (116.50 mmoles) of NMM and 11.5 ml (116.50 mmoles) of ECC were successively added at a temperature of -12 °C. After stirring at this temperature for 2 minutes, a cold solution of 28.3 g (116.50 mmoles) of HCl . H-Gly-OTce (II) and 12.9 ml (116.50 mmoles) of NMM in 100 ml of DMF was added. The reaction mixture was stirred for 1 hour at -12 °C, then filtered from salts and evaporated in vacuo. The residue was dissolved in AcOEt and washed several times with NaCl saturated solution. The organic layer was dried over anhydrous $Na_2SO_4$ and the solvent removed in vacuo. The crude residue was redissolved with $CH_2Cl_2$ and purified by column chromatography on silica gel (Merck) 0.040-0.063 mm, eluting with $CH_2Cl_2$/MeOH 99:1. After removal of the solvents, 51.1 g (86% yield) of compound III were obtained as an oil: $Rf_B$ = 0.79; $Rt_1$ = 17.8 minutes (linear gradient from 80 to 90% B in 15′, then isocratic 90% B for 10′).

Step 4 - HCl . H-Asp(OBzl)-Gly-OTce (IV)

Starting from 45.5 g (88.90 mmoles) of Boc-Asp(OBzl)-Gly-OTce (III), and operating as in Step 2, 39.2 g (98% yield) of compound IV were obtained as a foam: $Rf_A$ = 0,69.

Step 5 - Boc-Glu(OBzl)-Asp(OBzl)-Gly-OTce (V)

Starting from 29.5 g (87.47 mmoles) of Boc-Glu(OBzl)-OH and 39.2 g (87.47 mmoles) of HCl . H-Asp(OBzl)-Gly-OTce (IV), and operating as described in Step 3, 52.0 g (81% yield) of compound V were obtained as an oil: $Rf_B$ = 0.71.

Step 6 - HCl . H-Glu(OBzl)-Asp(OBzl)-Gly-OTce (VI)

Starting from 33.3 g (45.5 mmoles) of Boc-Glu(OBzl)-Asp(OBzl)-Gly-OTce (V), and operating as in Step 2, 29.5 g (98% yield) of compound VI were obtained as an oil: $Rf_A$ = 0.79.

Step 7 - Z-Leu-Glu(OBzl)-Asp(OBzl)-Gly-OTce (VII)

To a solution of 12.0 g (45.14 mmoles) of Z-Leu-OH in 100 ml of anhydrous THF, 5.0 ml of NMM (45.14 mmoles) and 4.5 ml (45.14 mmoles) of ECC were successively added at a temperature of -12 °C.
After stirring at this temperature for 2 minutes, a cold solution of 30.1 g (45.14 mmoles) of H-Glu(OBzl)-Asp-(OBzl)-Gly-OTce . HCl (VI) and 5.01 ml (45.14 mmoles) of NMM in 80 ml of DMF was added. The reaction mixture was stirred for 1 hour at -12 °C, then filtered from salts and evaporated in vacuo. The residue was dissolved in AcOEt and washed several times successively with NaCl saturated solutions of 0.5 M citric acid, 0.5 M $NaHCO_3$ and water. The organic layer was dried over anhydrous $Na_2SO_4$ and the solvent removed in vacuo.

36.7 g (92% yield) of compound VII were obtained from AcOEt/i-$Pr_2O$: m.p. = 109 (softening) -112 °C (dec.); $[\alpha]_D^{20}$ -16,8 ° (C 1, DMF); $Rf_B$ = 0.69; $Rt_3$ = 26.5 minutes (linear gradient from 70 to 90% B in 20′, then isocratic 90% B for 10′).

Step 8 - Z-Leu-Glu(OBzl)-Asp(OBzl)-Gly-OH (VIII)

A solution of 31.6 g (36.0 mmoles) of Z-Leu-Glu(OBzl)-Asp(OBzl)-Gly-OTce (VII) in 500 ml of AcOH and 215 ml of water was made to react at room temperature for 20 minutes with 31.6 g of zinc powder, washed with HCl 0.1 N, acetone and $Et_2O$. After filtration of the reaction mixture on hyflo supercell, the solvent was evaporated, the residue was dissolved in AcOEt and washed several times successively with NaCl saturated solutions of 0.5 M citric acid and water. The organic layer was dried over anhydrous $Na_2SO_4$ and the solvent removed in vacuo.
25.8 g (96% yield) of compound VIII were obtained from AcOEt/i-$Pr_2O$: m.p. = 158-164 °C (dec.); $[\alpha]_D^{20}$ = -19.8 ° (C 1, DMF); $Rf_B$ = 0.46; $Rt_2$ = 10.9 minutes (linear gradient from 70 to 90% B in 20′).

Step 9 - p-Tos-OH . H-Leu-OBzl (IX)

To a suspension of 13.12 g (100 mmoles) of H-Leu-OH in 105 ml of benzyl alcohol and 200 ml of anhydrous chloroform, 38.0 g (200 mmoles) of p-Tos-OH . $H_2O$ were added and the mixture was refluxed for 25 h. The water produced (5.4 ml) was collected using a Dean and Stark separator designed for use with solvents denser than water. Trituration with 500 ml of $Et_2O$ after cooling and evaporation of the chloroform gave 37.46 g (95% yield) of compound IX: m.p. 124-126 °C; $[\alpha]_D^{20}$ = -3.0 ° (C = 1, MeOH); $Rf_A$ = 0.65; $Rt_2$ = 16,7 minutes (linear gradient from 10 to 60% B in 20′).

Step 10 - Boc-Phe-Leu-OBzl (X)

To a solution of 41.52 g (156,5 mmoles) of Boc-Phe-OH in 240 ml of anhydrous THF, 17.36 ml (156.5 mmoles) of NMM and 15.36 ml (156.5 mmoles) of ECC were successively added at a temperature of -12 °C. After stirring at this temperature for 2 minutes, a cold solution of 61.52 g (156.5 mmoles) of P-Tos-OH . H-Leu-OBzl (IX) and 17.36 ml (156.5 mmoles) of NMM in 480 ml of DMF was added. The reaction mixture was stirred for 1 hour at -12 °C, then filtered from salts and evaporated in vacuo. The residue was dissolved in AcOEt and washed several times successively with NaCl saturated solutions of 0.5 M citric acid, 0.5 M $NaHCO_3$ and water. The organic layer was dried over anhydrous $Na_2SO_4$ and the solvent removed in vacuo.
69 g (94.1% yield) of compound X were obtained from AcOEt/light petroleum: m.p. 84-87 °C; $[\alpha]_D^{20}$ = -22.0 °C 1, MeOH); $Rf_B$ = 0.70; $Rt_5$ = 11.0 minutes (isocratic 90% B for 15′)

Step 11 - HCl . H-Phe-Leu-OBzl (XI)

32.0 g (68.3 mmoles) of Boc-Phe-Leu-OBzl (X) were dissolved in 320 ml of a saturated solution of HCl in AcOH. After 30 minutes at room temperature the Boc-removal was complete and the solvent was evaporated in vacuo at 30 °C.
26.0 g (94% yield) of compound XI were obtained from AcOEt: m.p. 158-160 C; $[\alpha]_D^{20}$ = -17.5 °(C 1, MeOH); $Rf_A$ = 0.72; $Rt_3$ = 13.8 minutes (linear gradient from 60 to 90% B in 20′).

Step 12 - Boc-Lys(Z)-Phe-Leu-OBzl (XII)

Starting from 24.37 g (64.1 mmoles) of HCl . H-Phe-Leu-OBzl (XI), and operating as in Step 10, 42.01 g (89.7% yield) of compound XII were obtained from AcOEt/$Et_2O$: m.p. 120-123 °C; $[\alpha]_D^{20}$ = -28.8 ° (C 1, MeOH); $Rf_B$ = 0.62; $Rt_3$ = 21.0 minutes (isocratic 90% B for 30′).

Step 13 - HCl . H-Lys(Z)-Phe-Leu-OBzl (XIII)

Starting from 41.96 g (57.4 mmoles) of Boc-Lys(Z)-Phe-Leu-OBzl (XII) and operating as described in Step 11, 36.82 g (96,1% yield) of compound XIII were obtained from MeOH/ACOEt: m.p. = 187-191 °C; $[\alpha]_D^{20}$ = -6.3 °C 1, MeOH); $Rf_A$ = 0.61; $Rt_3$ = 11.0 minutes (isocratic 90% B for 20′).

Step 14 - Boc-Pro-Lys(Z)-Phe-Leu-OBzl (XIV)

Starting from 1.08 g (5 mmoles) of Boc-Pro-OH and 3.34 g (5 mmoles) of HCl . H-Lys(Z)-Phe-Leu-OBzl (XIII) and operating as described in Step 10, 3.75 g (90% yield) of compound XIV were obtained from AcOEt/i-$Pr_2O$: m.p. = 124-8 °C; $[\alpha]_D^{20}$ = -47.9° (C 1, MEOH); $Rf_B$ = 0.64; $Rt_2$ = 19.4 minutes (linear gradient from 80 to 90% B in 20′, then isocratic 90% B for 5′).

Step 15 - HCl . H-Pro-Lys(Z)-Phe-Leu-OBzl (XV)

Starting from 3.75 g (4.5 mmoles) of Boc-Pro-Lys(Z)-Phe-Leu-OBzl (XIV) and operating as in Step 2, 3.2 g (91% yield) of compound XV were obtained from MeOH/AcOEt: m.p. = 100-103 °C; $[\alpha]_D^{20}$ = 33.8° (C 1, MeOH); $Rf_A$ = 0.58; $Rt_2$ = 7.5 minutes (linear gradient from 80 to 90% B in 20′).

Step 16 - Z-Leu-Glu(OBzl)-Asp(OBzl)Gly-Pro-Lys(Z)-Phe-Leu-OBzl (XVI)

A solution of 26.6 g (35.6 mmoles) of Z-Leu-Glu(OBzl)-Asp(OBzl)-Gly-OH (VIII) and 27.2 g (35.6 mmoles) of HCl . H-Pro-Lys(Z)-Phe-Leu-OBzl (XV) in 150 ml of DMF, were made to react for 4 hours at room temperature with 8.2 g (39.2 mmoles) of DCC, 5.3 g (39.2 mmoles) of anhydrous HOBt, 4.0 ml (35.6 mmoles) of NMM and 0.45 g (3.56 mmoles) of DMAP. The reaction mixture was then filtered from DCEU and concentrated to a small volume. The product was precipitated by dropwise addition of a cold aqueous solution of citric acid. 39.5 g (77% yield) of compound XVI were obtained from DMF/MeOH: m.p. = 179-82 °C (dec.); $[\alpha]_D^{20}$ = 35.5° (C 1, DMF); $Rf_B$ = 0.50; $Rt_4$ = 16.1 minutes (isocratic 100% B for 20′).

Step 17 - H-Leu-Glu-Asp-Gly-Pro-Lys-Phe-Leu-OH (XVII)

A solution of 31.5 g (21.6 mmoles) of Z-Leu-GlU(OBzl)-Asp(OBzl)-Gly-Pro-Lys(Z)-Phe-Leu-OBzl (XVI) in 330 ml of AcOH and 330 ml of MeOH was made to react for 20 minutes at room temperature with 25.8 g of 10% palladium on charcoal, 13.3 ml of formic acid, and 38.3 ml of NMM.
After filtration of the reaction mixture on hyflo supercell, the solvent was evaporated and 19.6 g (98% yield) of compound (XVII) were obtained from EtOH/AcOEt: m.p. = 206-210 °C (dec.); $[\alpha]_D^{20}$ = -44.7° (C 1, AcOH); $Rt_2$ = 12.0 minutes (linear gradient from 10 to 60% B in 20′)

Step 18 - H-Leu-Glu-Asp-Gly-Pro-Lys-Phe-Leu-OH . 2HCl (XVIII)

19.5 g (21.4 mmoles) of compound XVII were dissolved with 58.5 ml of HCl 1N and the solution was evaporated to residue. 20.6 g (97.2% yield) of compound XVIII were obtained from AcOH/$Et_2O$: m.p. = 108 °C (softening) -160 °C (dec.); $[\alpha]_D^{20}$ = -57,6° (C 0.5, MeOH); $Rt_2$ = 12.0 minutes (linear gradient from 10 to 60% B in 20′).

## Claims

**1.** A process for preparing a peptide of the formula (A)

H-Leu-Glu-Asp-Gly-Pro-Lys-Phe-Leu-OH (A)

or a pharmaceutically acceptable salt thereof, which process comprises condensing a compound of the formula (B)

```
          Y     Y'
          |     |
X-Leu-Glu-Asp-Gly-K      (B)
```

wherein X is an amino protecting group, Y and Y' each independently represents a carboxy protecting group and K is a hydroxy or hydrazido group, with a compound of formula (C):

W
|
H-Pro-Lys-Phe-Leu-Q (C)

wherein W is an amino protecting group and Q represents a carboxy protecting group or a hydroxy group, with the proviso that Q must be a carboxy protecting group when K is a hydroxy group; deprotecting the resultant compound of the formula (D)

Y Y' W
| | |
X-Leu-Glu-Asp-Gly-Pro-Lys-Phe-Leu-Q (D)

wherein X,Y,Y',W and Q are as defined above; and, if desired, converting the resulting peptide of formula (A) into a pharmaceutically acceptable salt thereof.

**2.** A process according to claim 1, wherein both Y and Y' groups are the same carboxy protecting group, Q represents a carboxy protecting group and K represents a hydroxy group.

**3.** A process according to claim 1 or 2, wherein the compound of formula (B) is the compound:

Z-Leu-Glu-(OBzl)-Asp(OBzl)-Gly-OH (VIII)

wherein Z represents benzyloxycarbonyl and Bzl represents benzyl, which has been obtained by treating with zinc powder a compound of the formula (VII):

Z-Leu-Glu(OBzl)-Asp(OBzl)-Gly-OTce (VII)

wherein Z and Bzl are as defined above and Tce represents 2,2,2-trichloroethyl.

**4.** A process according to any one of the preceding claims, further comprising formulating the peptide of formula (A) or pharmaceutically acceptable salt thereof thus produced with a pharmaceutically acceptable carrier or diluent.

**Patentansprüche**

**1.** Verfahren zur Herstellung eines Peptids der Formel (A)

H-Leu-Glu-Asp-Gly-Pro-Lys-Phe-Leu-OH (A)

oder eines pharmazeutisch akzeptablen Salzes desselben, wobei das Verfahren folgende Schritte umfaßt:

- Kondensieren einer Verbindung der Formel (B)

Y Y'
| |
X-Leu-Glu-Asp-Gly-K (B)

in der X eine Schutzgruppe für die Aminogruppe ist, Y und Y' jeweils unabhängig voneinander eine Schutzgruppe für die Carboxylgruppe darstellen, und K eine Hydroxy- oder Hydrazidogruppe ist, mit einer Verbindung der Formel (C)

W
|
H-Pro-Lys-Phe-Leu-Q (C)

in der W eine Schutzgruppe für die Aminogruppe ist, und Q eine Schutzgruppe oder eine Hydroxygruppe darstellt, unter der Voraussetzung, daß Q eine Schutzgruppe für die Carboxylgruppe sein muß, wenn K eine Hydroxygruppe ist;

- Entfernen der Schutzgruppen aus der resultierenden Verbindung der Formel (D)

```
          Y    Y'                W
          |    |                 |
X-Leu-Glu-Asp-Gly-Pro-Lys-Phe-Leu-Q          (D)
```

in der X, Y, Y', W und Q wie oben definiert sind; und, wenn gewünscht,

- Überführen des resultierenden Peptids der Formel (A) in ein pharmazeutisch akzeptables Salz des genannten.

**2.** Verfahren nach Anspruch 1, bei dem die Gruppen Y und Y' beide die gleichen Schutzgruppe für die Carboxylgruppe sind, Q eine Schutzgruppe für die Carboxylgruppe darstellt und K eine Hydroxygruppe darstellt.

**3.** Verfahren nach Anspruch 1 oder 2, bei dem die Verbindung der Formel (B) die Verbindung

Z-Leu-Glu(OBzl)-Asp(OBzl)-Gly-OH (VIII)

ist, worin Z für Benzyloxycarbonyl steht, und Bzl für Benzyl steht, welche durch Behandlung einer Verbindung der Formel (VII):

Z-Leu-Glu(OBzl)-Asp(OBzl)-Gly-OTce (VII)

worin Z und Bzl wie oben definiert sind, und Tce 2,2,2-Trichlorethyl darstellt, mit Zinkpulver erhalten wird.

**4.** Verfahren nach einem der vorangehenden Ansprüche, das außerdem die Formulierung des auf diese Weise hergestellten Peptids der Formel (A) oder eines pharmazeutisch akzeptablen Salzes davon mit einem pharmazeutisch akzeptablen Träger oder Verdünnungsmittel umfaßt.

## Revendications

**1.** Procédé de préparation d'un peptide représenté par la formule (A) :

H-Leu-Glu-Asp-Gly-Pro-Lys-Phe-Leu-OH (A)

ou d'un sel pharmaceutiquement acceptable dudit peptide, lequel procédé comprend :

- la condensation d'un composé de formule (B) :

```
          Y    Y'
          |    |
X-Leu-Glu-Asp-Gly-K          (B)
```

dans laquelle :

- X représente un groupe protecteur d'amino ;
- Y et Y' représentent chacun indépendamment un groupe protecteur de carboxy ; et
- K est un groupe hydroxy ou hydrazido,

avec un composé de formule (C) :

```
                W
                |
    H-Pro-Lys-Phe-Leu-Q              (C)
```

dans laquelle :
- W représente un groupe protecteur d'amino ; et
- Q représente un groupe protecteur de carboxy ou un groupe hydroxy,

avec la condition que Q doit être un groupe protecteur de carboxy lorsque K est un groupe hydroxy ;
- la déprotection du composé résultant de la formule (D):

```
            Y   Y'          W
            |   |           |
  X-Leu-Glu-Asp-Gly-Pro-Lys-Phe-Leu-Q      (D)
```

dans laquelle X, Y, Y', W et Q sont tels que définis ci-dessus; et
- si on le désire, la conversion du peptide résultant de la formule (A) en un sel pharmaceutiquement acceptable de celui-ci.

**2.** Procédé selon la revendication 1, dans lequel les groupes Y et Y' sont tous deux le même groupe protecteur de carboxy, Q représente un groupe protecteur de carboxy et K représente un groupe hydroxy.

**3.** Procédé selon la revendication 1 ou 2, dans lequel le composé de formule (B) est le composé :

Z-Leu-Glu-(OBzl)-Asp(OBzl)-Gly-OH (VIII)

dans laquelle :
- Z représente benzyloxycarbonyle ; et
- Bzl représente benzyle,

qui a été obtenu par traitement par de la poudre de zinc d'un composé de formule (VII) :

Z-Leu-Glu(OBzl)-Asp(OBzl)-Gly-OTce (VII)

dans laquelle :
- Z et Bzl sont tels que définis ci-dessus ; et
- Tce représente 2,2,2-trichloroéthyle.

**4.** Procédé selon l'une quelconque des revendications précédentes, comprenant en outre la formulation du peptide de formule (A) ou d'un sel pharmaceutiquement acceptable de celui-ci, ainsi obtenu, avec un support ou diluant pharmaceutiquement acceptable.